# EUROPEAN PATENT SPECIFICATION

(11) **EP 2 676 603 B1**
(45) Date of publication and mention of the grant of the patent: **25.03.2015**
(21) Application number: 12425113.3
(22) Date of filing: 18.06.2012
(51) Int. Cl.: A61B 5/0408, A61B 5/053, A61B 5/08, A61B 5/02

(54) **Sensor-enabled fabric label for detecting and transmitting electric signals or vital parameters of a user**
Sensor-aktiviertes Stoffetikett zum Erkennen und Übertragen von elektrischen Signalen oder Vitalparametern eines Anwenders
Étiquette de tissus avec capteur activé permettant de détecter et de transmettre des signaux électriques ou des paramètres vitaux d'un utilisateur

(43) Date of publication of application: 25.12.2013
(73) Proprietor: Comftech S.r.L., 20900 Monza (MB) (IT)
(72) Inventor: Andreoni, Giuseppe, 20835 Muggiò (MB) (IT); Moltani, Lara Alessia Laura, 20900 Monza (MB) (IT)
(74) Representative: Ciceri, Fabio

(56) References cited:
- EP-A2- 2 289 407
- WO-A2-03/079897
- US-A1- 2007 078 324
- US-A1- 2009 234 216

## Description

### FIELD OF THE INVENTION

The present invention relates to a sensor-enabled fabric label for detecting and transmitting electric signals or vital parameters of a user, particularly a fabric label that can be used, for instance, in the manufacture of clothing, medical devices and/or toys.

As used herein, the term labels is related to fabric labels of any one of the following classes:
1) fiber-based fabric labels, i.e. labels made of nonwoven fabric, such as felts, needlefelts and similar materials, which can be sewn and/or embroidered,
2) filament-based, warp-and-weft fabrics.

Il shall be noted that these labels may be loom-woven or thermocut, or defined by laser or ultrasounds.

### BACKGROUND ART

Sensor-enabled fabrics are known in the art, such as from document US 2007/0078324 and from document WO 03/079897.

For example, sensor-enabled fabrics are known, which consist of a fabric having a conductive fabric layer and an electric connector for connection to a processing device, e.g. external to the fabric.

A conductive paste is further known to be used to improve the electric signal detection performance of the sensor-enabled fabric.

Namely, such conductive paste is usually placed between the sensor and the electric connector but in certain applications, when the sensor-enabled fabric is implemented as a garment, the conductive paste may be incorporated in such garment in appropriate positions to obtain the desired electric signal.

electric signal detection, but introduces a number of problems for those who wear the sensor-enabled fabric.

For instance, the conductive paste shall be added every time that the garment incorporating the sensor-enabled fabric is worn, which will involve obvious and imaginable drawbacks in terms of time, costs and likelihood of staining the garments that contact the conductive paste.

Furthermore, the garment that incorporates the fabric with the conductive paste cannot be washed, because washing would affect or even hinder electric signal detection.

Furthermore, the use of a conductive paste in the sensor-enabled fabric is incompatible with the manufacture of garments suitable for immersion in liquids, such as bathing suits.

### SUMMARY OF THE PRESENT INVENTION

In view of the above prior art, the object of the present invention is to provide a device that can overcome the above mentioned drawbacks of the prior art.

This object is fulfilled by a sensor-enabled label, made of filament-based fabric, as defined in the features of claim 1.

Further embodiments are defined in dependent claims 2 - 13.

The present invention provides a sensor-enabled label that can detect electric signals representative of vital parameters of a user, such label being adapted to be integrated in a garment, a toy, and/or similar products.

The present invention allows the sensor-enabled label to be used in garments even when the wearer practices water sports, without affecting electric signal detection performance.

Furthermore, the present invention provides a label that can be used multiple times, without requiring any maintenance, and that can withstand multiple washing cycles in a washing machine.

### BRIEF DESCRIPTION OF THE FIGURES

The characteristics and advantages of the invention will appear from the following detailed description of one practical embodiment, which is illustrated without limitation in the annexed drawings, in which:
- Figure 1 is a schematic exploded view of a label according to one embodiment,
- Figure 2A and Figure 2B show top and bottom views, respectively, of a label according to one embodiment,
- Figure 3A and Figure 3B show top and bottom views, respectively, of a possible example of the label of Figures 2A and 2B,
- Figure 3C shows a possible way of use of the label of Figures 3A and 3B, when it is associated with an electronic processing device,
- Figures 4A and 4B show top and bottom views, respectively, of a label according to another embodiment,
- Figures 5 and 6 show possible ways of use of the label as shown in Figure 1.

### DETAILED DISCLOSURE OF THE INVENTION

Referring to the accompanying figures, numeral 1 shows a sensor-enabled label.

In the present invention, the sensor-enabled label 1 is made of filament-based fabric, e.g. a warp-and-weft fabric.

For this purpose, feasible fabrics may include openwork braids, orthogonal fabrics, knitted, plain weave, twill weave fabrics or generally jacquard and embroidered fabrics.

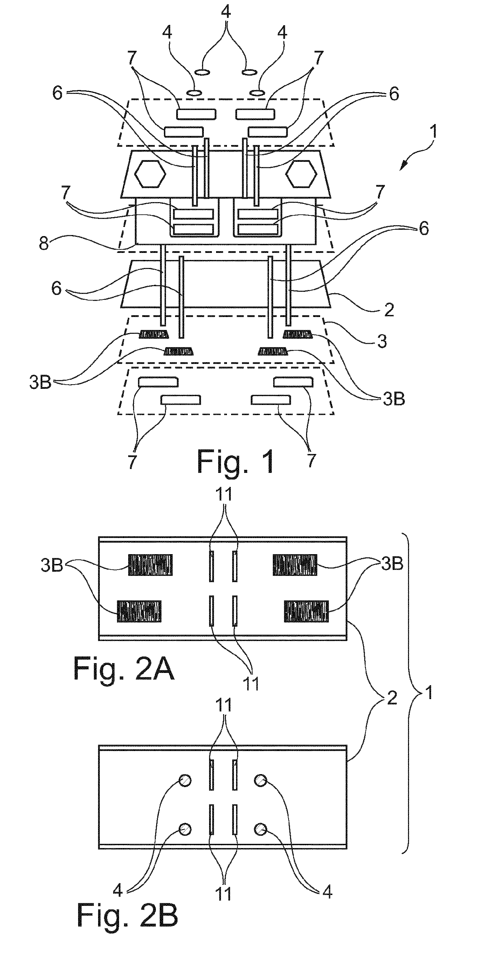

Referring to Figure 1, it shall be noted that, for simplicity, the sensor-enabled label 1 is shown as consisting of three layers, although such layers are woven in the fabric that forms the sensor-enabled label.

The sensor-enabled label 1 is designed to detect and transmit electric signals, e.g. vital parameters such as cardiac signals, respiratory signals, etc. of a user once the label has contacted the user's skin.

The sensor-enabled label 1 has ergonomic properties such as breathability, perspiration resistance, washability and anthropometric fit in terms of sizing.

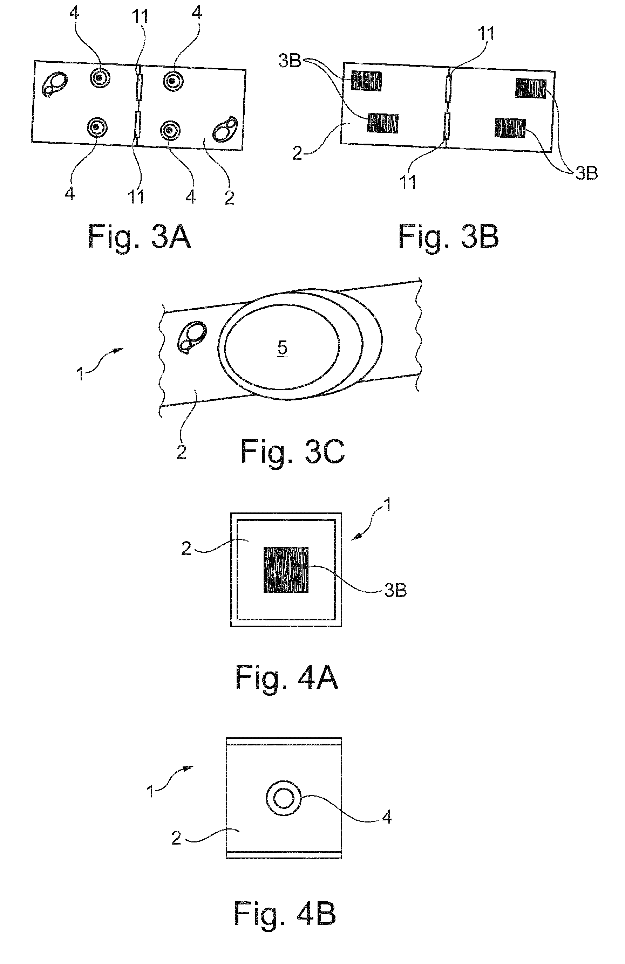

For this purpose, the sensor-enabled label 1 comprises:
- a fabric 2;
- a layer of conductive fabric 3 integrated in the fabric 2;
- at least one electric connector 4 for connection to a processing device 5 (as shown in Figure 3C),
- the at least one electric connector 5 being in signal communication with the layer of conductive fabric 3 through an electrical connection 6.

It shall be noted that the layer of conductive fabric 3 comprises fibers which are already electrically conductive before being incorporated in the fabric 2, i.e. fibers having original electrical conductivity properties.

These electrically conductive fibers are woven or sewn into the fabric 2, and form, for instance, a part of the warp, the weft, or both of the fabric 2.

In one aspect, the electrically conductive fibers consist of silver, steel, carbon, copper wires or fibers coated with an electrically conductive material, or other materials having similar electrical conductivity.

It shall be noted that, upon incorporation of the electrically conductive fibers into the fabric 2, a plurality of transducers 3B may be provided on one side of the label 1, i.e. the side that is designed to contact the user's skin.

Such transducers 3B actually act as sensors for detecting electric signals generated (e.g. ECG signals) or modulated (e.g. bioimpedance signals) by the body of the user with which the label 1 is associated.

Therefore, the transducers 3B can detect vital parameters of the user (i.e. the electric signals) and transmit them to the electric connectors 4.

In other words, the plurality of transducers 3B are visible by the user when the label 1 is not being used.

Such transducers 3B may have a surface area ranging from some fraction of the overall area of the side of the fabric 2 that is designed to contact the user's skin to the entire or total area of the side of the fabric that is designed to contact the user's skin.

For example, the plurality of transducers 3B may cover about 10% of the entire surface area of the side of the fabric 2 that is designed to contact the user's skin.

The surface area of transducers 3B shall be selected according to the application for which the sensor-enabled label 1 is designed.

In one aspect, each transducer of the plurality of transducers 3B may have a particular surface area, that may be equal to or different from that of the other transducers.

In one aspect, the plurality of transducers 3B provide a number equal to or greater than one, preferably two, three, four, five, six, seven and eight detection points.

In other words, the transducers 3B consist in portions of the fabric 2 that are composed of a plurality of electrically conductive fibers with a density equal to or higher than the density with which the fabric 2 is made.

As shown in Figures 2A and 4A, the transducers 3B are electrically conductive portions or pads of the fabric 2, raised relative to the free surface of the fabric 2.

The electric connector 4 is, for instance, either a snap connector, or a bayonet, strip, or fast-on connector.

These electric connectors 4 are placed on the side opposite to the side on which the plurality of transducers 3B are located.

In other words, the sensor-enabled label 1 defines two larger sides, preferably flat and parallel to each other, i.e. one that holds the plurality of transducers 3B (i.e. the side that contacts the user's skin) and one that holds the connectors 4.

In order to transfer the relevant vital parameter from the transducer 3B to the connector 4, an electrical connection 6 is provided, that can convey the electric signal detected by the transducers 3B.

Such electrical connection 6 consists, for instance, of an electrically conductive wire, such as a silver, steel, carbon, copper wire or fibers coated with metals or conductive materials, or materials having similar electrical conductivity.

The electrical connection 6 is woven into the fabric 2.

The processing device 5 is an electronic device designed to process the signals detected by the transducers 3B and transferred through the electrical connections 6 to the electric connectors 4.

Such processing device 5 consists, for instance of a single- or multi-lead electrocardiograph, an ECG holter, a heart rate monitor, a multi-parameter polygraph or actigraph, a bioimpedance-based meter for measuring respiratory parameters, a textile or non-textile strain gauge.

In one aspect, the processing device 5 may be integrated in the fabric 2 itself or be preferably located outside the fabric 2.

In one aspect, the fabric 2 of the sensor-enabled label 1 comprises elements that characterize its ergonomic properties, such as the breathability imparted to the fabric, its flexibility and softness for improved anthropometric fit and at least one slot 11, i.e. a through hole that extends through the entire thickness of the fabric 2.

The sensor-enabled label 1 comprises an impedance adapter 7.

The impedance adapter 7 is designed to modulate its impedance to match the bioimpedance value exhibited by the user, and hence optimize the interface between his/her skin and the plurality of transducers 3B.

In other words, the impedance adapter 7 is designed to adapt the impedance value generated when the label contacts the user's skin, i.e. between the plurality of transducers 3B and the skin.

This will afford improved electric signal detection by the transducers 3B, as compared with prior art devices.

In one aspect, the impedance adapter 7 is associated with the layer of conductive fabric 3.

Preferably, the impedance adapter 7 is interposed between the user's skin and the layer of conductive fabric 3.

Namely, the impedance adapter 7 is associated and in contact with the layer of conductive fabric 3.

Alternatively, as shown in Figure 1, the impedance adapter 7 is interposed between the fabric 2 and the at least one electric connector 4.

In one aspect, the impedance adapter 7 may be arranged to be interposed both between the user's skin and the layer of conductive fabric 3 and between the fabric 2 and the at least one electric connector 4.

It shall be noted that, in one embodiment, the impedance adapter 7 comprises a layer of conductive rubber, which is located proximate to each transducer 3B.

Particularly, this rubber layer may have a thickness ranging from 0.2 mm to 7 mm, and a surface area equal to the surface area of the conductive layer 3, i.e. equal to the surface area of the side of the fabric 2 that contacts the user's skin.

For example, the rubber layer 7 may be the one sold by B.C.E. srl, based in Sassuolo (MO), Via Regina Pacis 54/c 41049, or by Soliani Emc srl, based in Como.

In one aspect, the rubber layer of the impedance adapter 7 is located in the proximity of each transducer 3B and has a surface area equal to or preferably larger than the surface area of each transducer 3B.

Particularly, the surface area of the rubber layer of the impedance adapter 7 shall be large enough to optimize the impedance value (or interface) generated between the user's skin and the plurality of transducers 3B.

If the impedance adapter 7 is also interposed between the fabric 2 and the at least one electric connector 4, then the surface area of the rubber layer is equal to, preferably larger than, the area of the electric connectors 4.

Alternatively, the impedance adapter 7 includes a textile yarn having an electrical conductivity (about 10-500 Ohm) lower than the electrical conductivity of the layer of conductive fabric of the transducers 3B.

Also in this embodiment, the surface area of the textile yarn having an electrical conductivity lower than the electrical conductivity of the layer of conductive fabric of the transducers 3B is equal to the surface area of each transducer 3B and, if the impedance adapter 7 is also interposed between the fabric 2 and the at least one electric connector 4, then the surface area of the yarn is equal to, or preferably larger than the area of the electric connectors 4.

The impedance adapter 7 comprises an electronic impedance-matching circuit 8.

This electronic impedance-matching circuit 8 is designed to modulate its impedance to match the bioimpedance value exhibited by the user.

In one aspect the sensor-enabled label 1 may be formed as a patch, i.e. a plasticized strip having adhesive to adhere to the user's skin.

The adhesive is placed on the side of the fabric 2 having the plurality of transducers 3B.

It shall be noted that the slots 7 afford improved transpiration from the skin of the patch wearer, thereby preventing sweating, that might cause alterations in the detection of vital parameters by the transducers 3B and/or peeling off of the label and/or irritation or damages to the individual's skin.

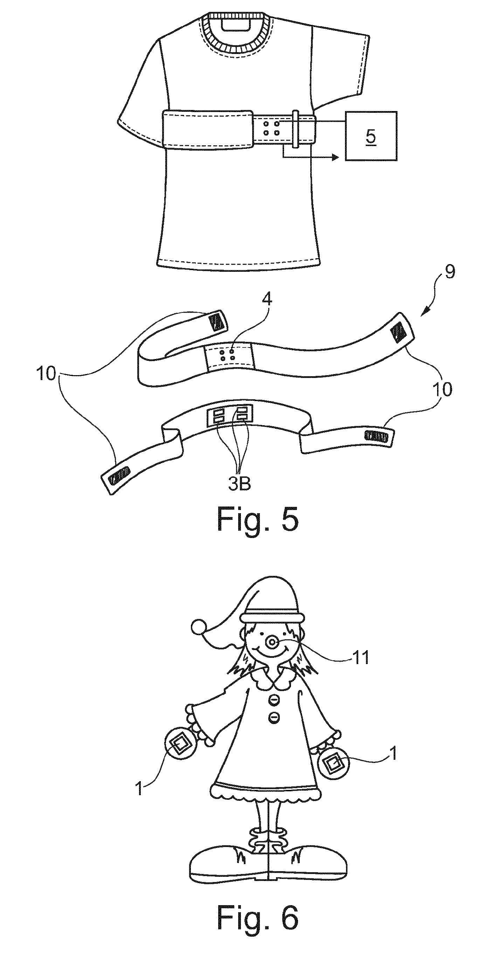

Referring now to Figure 5, a possible way of use of the sensor-enabled label 1 is shown, which label is integrated, for instance, in a garment, namely consisting of a chest strap 9.

Such chest strap 9 has fastening means 10 to enclose the user's chest and can support the processing device 5, to process the detected signals without requiring any additional electronic instrument.

Referring now to Figure 6, another possible way of use of the sensor-enabled label 1 is shown, which label is integrated, for instance, in a toy.

In this implementation, the human body contacts the sensor-enabled label 1 through the plurality of transducers 3B located on the surface of the toy (as shown in the figure next to the hand palms) to actuate, for instance, a LED 11 upon processing by the processing device 5 integrated in the sensor-enabled label 1.

Those skilled in the art will obviously appreciate that a number of changes and variants may be made to the arrangements as described hereinbefore to meet specific needs, without departure from the scope of the invention, as defined in the following claims.

## Claims

1. A sensor-enabled fabric label (1) for detecting and transmitting electric signals or vital parameters of a user, comprising:
- a filament-based fabric (2);
- a layer of conductive fabric (3) integrated in said filament-based fabric (2), to define a plurality of electric signal transducers (3B);
- at least one electric connector (4) for connection to a processing device (5), said at least one electric connector (4) being in signal communication with said layer of conductive fabric (3) through an electrical connection (6);
- an impedance adapter (7) designed to match the impedance value between the plurality of transducers (3B) and the user's skin, when the sensor-enabled label (1) contacts the user's skin,
**characterized in that** said impedance adapter (7) is interposed between said fabric (2) and said at least one electric connector (4), said at least one electric connector (4) being placed on said filament-based fabric (2) on the side opposite to the side on which the plurality of said electric signal transducers (3B) are located, said impedance adapter (7) comprising an electronic impedance-matching circuit (8) designed to modulate its impedance to match the bio impedance value exhibited by the user.

2. A sensor-enabled fabric label as claimed in claim 1, wherein said impedance adapter is associated and in contact with said plurality of electric signal transducers (3B).

3. A sensor-enabled fabric label as claimed in claim 1 or 2, wherein said impedance adapter (7) is associated with said layer of conductive fabric (3) by being interposed between the user's skin and said layer of conductive layer (3).

4. A sensor-enabled fabric label as claimed in claim 1, wherein said impedance adapter (7) comprises a layer of conductive rubber having a thickness ranging from 0.2 mm to 7 mm, and a surface area equal to or larger than the surface area of said layer of conductive fabric (3) of said plurality of transducers (3B) or said at least one electric connector (4).

5. A sensor-enabled fabric label as claimed in claim 1, wherein said impedance adapter (7) comprises a textile yarn having an electrical conductivity lower than the electrical conductivity of said layer of conductive fabric (2).

6. A sensor-enabled fabric label as claimed in any preceding claim, wherein said layer of conductive fabric (2) comprises electrically conductive fibers, which are electrically conductive prior to their incorporation in said fabric, said electrically conductive fibers being sewn or woven into said fabric (2) to form said plurality of electric signal transducers (3B).

7. A sensor-enabled fabric label as claimed in claim 1, wherein said plurality of electric transducers (3B) consist of electrically conductive portions or pads of the fabric (2).

8. A sensor-enabled label as claimed in claim 1, wherein said plurality of electric transducers (3B) have a surface area ranging from a fraction of the surface area of the fabric (2) to the entire or whole surface area of the fabric (2) designed to be contacted by the user's skin.

9. A sensor-enabled fabric label as claimed in claim 1, wherein said at least one electric connector (4) is either a snap connector, or a bayonet, strip, or fast-on connector.

10. A sensor-enabled fabric label as claimed in claim 1, wherein said fabric has at least one slot (11).

11. A sensor-enabled fabric label as claimed in claim 1, wherein said layer of conductive fabric (3) consist of silver, steel, carbon, copper wires or fibers coated with an electrically conductive material, or other materials having similar electrical conductivity.

12. A garment comprising at least one sensor-enabled fabric label as claimed in any claim from 1 to 11.

13. A toy comprising at least one sensor-enabled fabric label as claimed in any claim from 1 to 11.

## Patentansprüche

1. Sensor-aktiviertes Stoffetikett (1) zum Erkennen und Übertragen von elektrischen Signalen oder Vitalparametern eines Anwenders, umfassend:
- ein Filament-basienes Gewebe (2);
- eine Lage aus leitfähigem Gewebe (3), die in das Filament-basierte Gewebe (2) integriert ist, um eine Vielzahl von elektrischen Signalwandlern (3B) zu definieren;
- mindestens einen elektrischen Verbinder (4) für die Verbindung mit einer Verarbeitungseinrichtung (5), wobei der mindestens eine elektrische Verbinder (4) durch eine elektrische Verbindung (6) in einer Signalverbindung mit der Lage aus leitfähigem Gewebe (3) steht;
- eine Impedanzanpassungseinrichtung (7), die gestaltet ist, um den Impedanzwert zwischen den vielen Wandlern (3B) und der Haut des Anwenders anzupassen, wenn das Sensor-aktivierte Etikett (1) die Haut des Anwenders berührt,
**dadurch gekennzeichnet**, **dass** die Impedanzanpassungseinrichtung (7) zwischen das Gewebe (2) und den mindestens einen elektrischen Verbinder (4) eingefügt ist, wobei der mindestens eine elektrische Verbinder (4) auf dem Filament-basierten Gewebe (2) auf der Seite angeordnet ist, die der Seite entgegengesetzt ist, auf der sich die Vielzahl von elektrischen Signalwandlern (3B) befindet, wobei die Impedanzanpassungseinrichtung (7) eine elektronische Impedanzanpassungsschaltung (8) umfasst, die gestaltet ist, um ihre Impedanz zum Anpassen an den Bio-Impedanzwert, den der Anwender aufweist, zu modulieren.

2. Sensor-aktiviertes Stoffetikett nach Anspruch 1, bei dem die Impedanzanpassungseinrichtung mit den vielen elektrischen Signalwandlern (3B) verbunden und mit ihnen in Kontakt ist.

3. Sensor-aktiviertes Stoffetikett nach Anspruch 1 oder 2, bei dem die Impedanzanpassungseinrichtung (7) mit der Lage aus leitfähigem Gewebe (3) verbunden ist, indem sie zwischen die Haut des Anwenders und die Lage aus einer leitfähigen Schicht (3) eingefügt ist.

4. Sensor-aktiviertes Stoffetikett nach Anspruch 1, bei dem die Impedanzanpassungseinrichtung (7) eine Leitgummischicht mit einer Dicke im Bereich von 0,2 mm bis 7 mm und mit einem Flächeninhalt, der gleich oder größer als der Flächeninhalt der Lage aus leitfähigem Gewebe (3) der Vielzahl von Wandlern (3B) oder des mindestens einen elektrischen Verbinders (4) ist, umfasst.

5. Sensor-aktiviertes Stoffetikett nach Anspruch 1, bei dem die Impedanzanpassungseinrichtung (7) ein textiles Garn mit einer elektrischen Leitfähigkeit umfasst, die geringer als die elektrische Leitfähigkeit der Lage aus leitfähigem Gewebe (2) ist.

6. Sensor-aktiviertes Stoffetikett nach einem der vorhergehenden Ansprüche, bei dem die Lage aus leitfähigem Gewebe (2) elektrisch leitfähige Fasern umfasst, die vor ihrer Einarbeitung in dieses Gewebe elektrisch leitfähig sind, wobei diese elektrisch leitfähigen Fasern in das Gewebe (2) eingenäht oder eingewoben sind, um die Vielzahl von elektrischen Signalwandlern (3B) auszubilden.

7. Sensor-aktiviertes Stoffetikett nach Anspruch 1, bei dem die Vielzahl von elektrischen Wandlern (3B) aus elektrisch leitfähigen Abschnitten oder Feldern des Gewebes (2) besteht.

8. Sensor-aktiviertes Etikett nach Anspruch 1, bei dem die Vielzahl von elektrischen Wandlern (3B) einen Flächeninhalt hat, der im Bereich von einem Bruchteil des Flächeninhalts des Gewebes (2) bis zum gesamten oder ganzen Flächeninhalt des Gewebes (2) liegt, das gestaltet ist, um von der Haut des Anwenders berührt zu werden.

9. Sensor-aktiviertes Stoffetikett nach Anspruch 1, bei dem der mindestens eine elektrische Verbinder (4) entweder ein Schnappverbinder oder ein Bajonett-, Leisten- oder Faston-Verbinder ist.

10. Sensor-aktiviertes Stoffetikett nach Anspruch 1, bei dem das Gewebe mindestens einen Schlitz (11) aufweist.

11. Sensor-aktiviertes Stoffetikett nach Anspruch 1, bei dem die Lage aus leitfähigem Gewebe (3) aus Silber-, Stahl-, Kohle-, Kupferdrähten oder Fasern, die mit einem elektrisch leitfähigen Material beschichtet sind, oder anderen Materialien mit einer vergleichbaren elektrischen Leitfähigkeit besteht.

12. Kleidungsstück, das mindestens ein Sensor-aktiviertes Stoffetikett nach einem der Ansprüche von 1 bis 11 umfasst.

13. Spielzeug, das mindestens ein Sensor-aktiviertes Stoffetikett nach einem der Ansprüche von 1 bis 11 umfasst.

## Revendications

1. Etiquette de tissu avec capteur activé (1) pour détecter et transmettre des signaux électriques ou des paramètres vitaux d'un utilisateur, comprenant :
- un tissu à base de filaments (2) ;
- une couche de tissu conducteur (3) intégrée dans ledit tissu à base de filaments (2), pour définir une pluralité de transducteurs de signal électrique (3B) ;
- au moins un connecteur électrique (4) pour la connexion à un dispositif de traitement (5), ledit au moins un connecteur électrique (4) étant en communication de signal avec ladite couche de tissu conducteur (3) à travers une connexion électrique (6) ;
- un adaptateur d'impédance (7) conçu pour adapter la valeur d'impédance entre la pluralité de transducteurs (3B) et la peau de l'utilisateur, quand l'étiquette avec capteur activé (1) est en contact avec la peau de l'utilisateur ;
**caractérisée en ce que** ledit adaptateur d'impédance (7) est interposé entre ledit tissu (2) et ledit au moins un connecteur électrique (4), ledit au moins un connecteur électrique (4) étant placé sur ledit tissu à base de filaments (2) sur le côté opposé au côté sur lequel est située la pluralité destits transducteurs de signal électrique (3B), ledit adaptateur d'impédance (7) comprenant un circuit d'adaptation d'impédance électronique (8) conçu pour moduler son impédance pour s'adapter à la valeur de bio-impédance présentée par l'utilisateur.

2. Etiquette de tissu avec capteur activé selon la revendication 1, dans laquelle ledit adaptateur d'impédance est associé et en contact avec ladite pluralité de transducteurs de signal électrique (3B).

3. Etiquette de tissu avec capteur activé selon la revendication 1 ou 2, dans laquelle ledit adaptateur d'impédance (7) est associé à ladite couche de tissu conducteur (3) en étant interposé entre la peau de l'utilisateur et ladite couche de couche conductrice (3).

4. Etiquette de tissu avec capteur activé selon la revendication 1, dans laquelle ledit adaptateur d'impédance (7) comprend une couche de caoutchouc conducteur ayant une épaisseur dans la plage de 0,2 mm à 7 mm et une superficie égale ou supérieure à la superficie de ladite couche de tissu conducteur (3) de ladite pluralité de transducteurs (3B) ou dudit au moins un connecteur électrique (4).

5. Etiquette de tissu avec capteur activé selon la revendication 1, dans laquelle ledit adaptateur d'impédance (7) comprend un filé textile ayant une conductivité électrique inférieure à la conductivité électrique de ladite couche de tissu conducteur (2).

6. Etiquette de tissu avec capteur activé selon une quelconque revendication précédente, dans laquelle ladite couche de tissu conducteur (2) comprend des fibres électriquement conductrices, qui sont électriquement conductrices avant leur incorporation dans ledit tissu, lesdites fibres électriquement conductrices étant cousues ou tissées dans ledit tissu (2) pour former ladite pluralité de transducteurs de signal électrique (3B).

7. Etiquette de tissu avec capteur activé selon la revendication 1, dans laquelle ladite pluralité de transducteurs électriques (3B) comprend des portions ou coussinets électriquement conducteurs du tissu (2).

8. Etiquette avec capteur activé selon la revendication 1, dans laquelle ladite pluralité de transducteurs électriques (3B) a une superficie allant d'une fraction de la superficie du tissu (2) à la totalité de la superficie du tissu (2) destiné à être touché par la peau de l'utilisateur.

9. Etiquette de tissu avec capteur activé selon la revendication 1, dans laquelle ledit au moins un connecteur électrique (4) est soit un connecteur à encliquetage, soit un connecteur à baïonnette, à bande ou rapide.

10. Etiquette de tissu avec capteur activé selon la revendication 1, dans laquelle ledit tissu comporte au moins une fente (11).

11. Etiquette de tissu avec capteur activé selon la revendication 1, dans laquelle ladite couche de tissu conducteur (3) est constituée par des fils ou fibres d'argent, d'acier, de carbone, de cuivre, revêtus avec un matériau électriquement conducteur, ou d'autres matériaux ayant une conductivité électrique similaire.

12. Vêtement comprenant au moins une étiquette de tissu avec capteur activé selon une quelconque revendication 1 à 11.

13. Jouet comprenant au moins une étiquette de tissu avec capteur activé selon une quelconque revendication 1 à 11.
